(19) Europäisches Patentamt
European Patent Office
Office européen des brevets

(11) Veröffentlichungsnummer: **0 171 729**
**A1**

(12) # EUROPÄISCHE PATENTANMELDUNG

(21) Anmeldenummer: 85109817.8

(22) Anmeldetag: 05.08.85

(51) Int. Cl.⁴: C 07 D 401/04
A 01 N 43/56, A 01 N 43/653
A 01 N 43/50

(30) Priorität: 17.08.84 DE 3430232

(43) Veröffentlichungstag der Anmeldung:
19.02.86 Patentblatt 86/8

(84) Benannte Vertragsstaaten:
CH DE FR GB IT LI

(71) Anmelder: BAYER AG
Konzernverwaltung RP Patentabteilung
D-5090 Leverkusen 1 Bayerwerk(DE)

(72) Erfinder: Knops, Hans-Joachim, Dr.
Koepenickerstrasse 35
D-4019 Monheim(DE)

(72) Erfinder: Babczinski, Peter, Dr.
In der Lohrenbeck 11
D-5600 Wuppertal 1(DE)

(72) Erfinder: Eue, Ludwig, Dr.
Paul-Klee-Strasse 36
D-5090 Leverkusen 1(DE)

(72) Erfinder: Santel, Hans-Joachim, Dr.
Gerstenkamp 19
D-5000 Koeln 80(DE)

(72) Erfinder: Schmidt, Robert R. Dr.
Im Waldwinkel 110
D-5060 Bergisch-Gladbach 2(DE)

(54) **N-Methyl-4-pyridone.**

(57) Die Erfindung betrifft neue N-Methyl-4-pyridone der Formel (I)

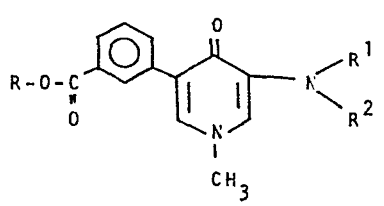

(I)

in welcher

R für Wasserstoff, Alkyl, Alkenyl, Alkinyl, Alkoxyalkyl, Halogenalkyl, Cycloalkyl, für gegebenenfalls substituiertes Aralkyl oder für ein Äquivalent eines Ammonium- oder Metallkations steht und

$R^1$ und $R^2$ gemeinsam mit dem Stickstoffatom, an welches sie gebunden sind, für einen gegebenenfalls substituierten Heterocyclus stehen,

Verfahren zu ihrer Herstellung und ihre Verwendung als Herbizide.

BAYER AKTIENGESELLSCHAFT
Konzernverwaltung RP
Patentabteilung

5090 Leverkusen, Bayerwerk
KM/Ja-c
Ib

## N-Methyl-4-pyridone

Die Erfindung betrifft neue N-Methyl-4-pyridone, mehrere Verfahren zu ihrer Herstellung und ihre Verwendung als Herbizide.

Es ist bereits bekannt, daß bestimmte N-substituierte 4-Pyridone, wie beispielsweise das 1-Methyl-3-(1,2,4-triazol-1-yl)-5-(3-trifluormethylphenyl)-4-pyridon, herbizide Eigenschaften, insbesondere auch selektiv-herbizide Eigenschaften besitzen (vergl. z.B. EP-A2-0 073 999 und US-PS 4 451 282).

Die herbizide Wirkung dieser vorbekannten Verbindungen gegenüber Schadpflanzen, ebenso wie ihre Verträglichkeit gegenüber Kulturpflanzen, ist jedoch nicht immer in allen Anwendungsbereichen völlig zufriedenstellend.

Es wurden neue N-Methyl-4-pyridone der allgemeinen Formel (I),

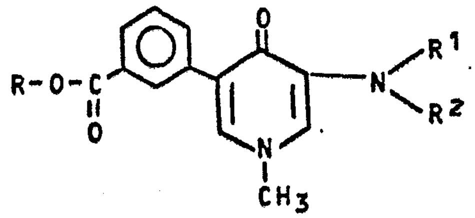

(I)

in welcher

R für Wasserstoff, Alkyl, Alkenyl, Alkinyl, Alkoxyalkyl, Halogenaklyl, Cycloalkyl, für gegebenenfalls substituiertes Aralkyl oder für ein Äquivalent eines Ammonium- oder Metallkations steht und

$R^1$ und $R^2$ gemeinsam mit dem Stickstoffatom, an welches sie gebunden sind, für einen gegebenenfalls substituierten Heterocyclus stehen,

gefunden.

Weiterhin wurde gefunden, daß man die neuen N-Methyl-4-pyridone der allgemeinen Formel (I) erhält, wenn man

(a) Trifluormethylphenyl-pyridone der Formel (II),

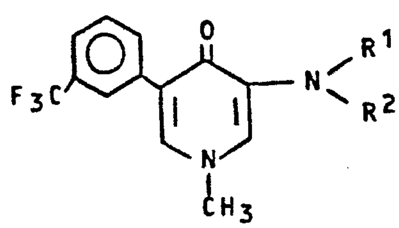

(II)

in welcher

$R^1$ und $R^2$ die oben angegebene Bedeutung haben,

mit konzentrierter Schwefelsäure verseift, oder wenn man

(b) die nach Verfahren (a) erhältlichen Carbonsäuren der Formel (Ia)

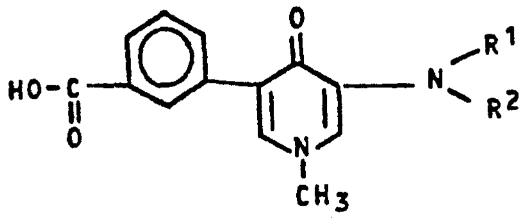

(Ia)

in welcher

$R^1$ und $R^2$ die oben angegebene Bedeutung haben,

mit Hydroxyverbindungen der Formel (III),

$$R' - OH \qquad (III)$$

in welcher

R' für Alkyl, Alkenyl, Alkinyl, Alkoxyalkyl, Halogenalkyl, Cycloalkyl, für gegebenenfalls substituiertes Aralkyl oder für ein Äquivalent eines Metallkations oder quarternären Ammoniumions steht,

oder mit Aminen der Formel (IV)

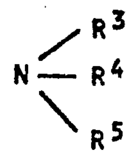 (IV)

in welcher

$R^3$, $R^4$ und $R^5$ unabhängig voneinander jeweils für Wasserstoff oder für Alkyl, Cycloalkyl oder Aralkyl stehen,

gegebenenfalls in Gegenwart eines Verdünnungsmittels und gegebenenfalls in Gegenwart eines Katalysators umsetzt, oder wenn man

(c) die nach Verfahren (b) erhältlichen Ester der Formel (Ib),

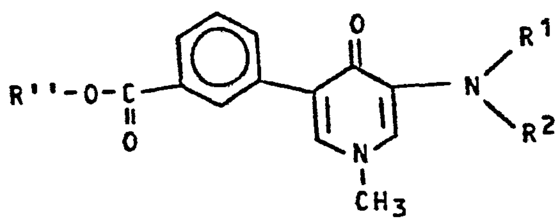 (Ib)

in welcher

R'' für niederes Alkyl steht

und

$R^1$ und $R^2$ die oben angegebene Bedeutung haben,

mit Alkoholen der Formel (IIIa)

$$R''' - OH \qquad (IIIa)$$

in welcher

R''' für Alkyl, Alkenyl, Alkinyl, Alkoxyalkyl, Halogenalkyl, Cycloalkyl oder für gegebenenfalls substituiertes Aralkyl steht,

gegebenenfalls in Gegenwart eines Verdünnungsmittels und gegebenenfalls in Gegenwart eines Katalysators umsetzt.

Schließlich wurde gefunden, daß die neuen N-Methyl-4-pyridone der allgemeinen Formel (I) herbizide, insbesondere auch selektiv-herbizide Eigenschaften besitzen.

Überraschenderweise zeigen die erfindungsgemäßen N-Methyl-4-pyridone der allgemeinen Formel (I) bei vergleichbarer Nutzpflanzenselektivität eine erheblich bessere herbizide Wirksamkeit gegenüber Schadpflanzen, als die aus dem Stand der Technik bekannten N-substituierten 4-Pyridone, wie beispielsweise das 1-Methyl-3-(1,2,4-triazol-1-yl)-5-(3-trifluormethylphenyl)-4-pyridon, welches chemisch und wirkungsmäßig naheliegende Verbindungen sind.

Die erfindungsgemäßen N-Methyl-4-pyridone sind durch die Formel (I) allgemein definiert. Bevorzugt sind Verbindungen der Formel (I), bei welchen

R für Wasserstoff, für jeweils geradkettiges oder

verzweigtes Alkyl, Alkenyl oder Alkinyl mit jeweils bis zu 18 Kohlenstoffatomen, für geradkettiges oder verzweigtes Alkoxyalkyl mit jeweils bis zu 12 Kohlenstoffatomen in den einzelnen Alkylteilen, für geradkettiges oder verzweigtes Halogenalkyl mit bis zu 12 Kohlenstoffatomen und bis zu 18 gleichen oder verschiedenen Halogenatomen, für Cycloalkyl mit 3 bis 7 Kohlenstoffatomen oder für gegebenenfalls einfach oder mehrfach, gleich oder verschieden im Arylteil substituiertes, geradkettiges oder verzweigtes Aralkyl mit bis zu 4 Kohlenstoffatomen im Alkylteil und 6 bis 10 Kohlenstoffatomen im Arylteil steht, wobei als Substituenten infrage kommen: Halogen, Cyano, Nitro, sowie jeweils geradkettiges oder verzweigtes Alkyl, Alkoxy oder Halogenalkyl mit jeweils bis zu 4 Kohlenstoffatomen und gegebenenfalls bis zu 9 gleichen oder verschiedenen Halogenatomen, außerdem für ein Äquivalent eines Alkali- oder Erdalkalimetallkations oder für ein Ammoniumion der Formel

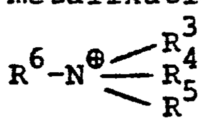

steht, wobei $R^3$, $R^4$, $R^5$ und $R^6$ unabhängig voneinander jeweils für Wasserstoff, für geradkettiges oder verzweigtes Alkyl mit bis zu 18 Kohlenstoffatomen, für Cycloalkyl mit 3 bis 7 Kohlenstoffatomen oder für geradkettiges oder verzweigtes Aralkyl mit bis zu 4 Kohlenstoffatomen im Alkylteil und 6 bis 10 Kohlenstoffatomen im Arylteil stehen,

und

$R^1$ und $R^2$ gemeinsam mit dem Stickstoffatom, an welches sie gebunden sind, für einen gegebenenfalls durch Alkyl oder Alkoxy mit jeweils bis zu 4 Kohlenstoffatomen oder Halogen substituierten 5-gliedrigen Heterocyclus mit 1 bis 3 Stickstoffatomen stehen.

Besonders bevorzugt sind Verbindungen der Formel (I), bei welchen

R für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, n- oder i-Nonyl, n- oder i-Decyl, n- oder i-Dodecyl, Allyl, Butenyl, Pentenyl, Hexenyl, Propargyl, Butinyl, Pentinyl, Hexinyl, Methoxymethyl, Methoxyethyl, Ethoxyethyl, Isopropoxyethyl, Methoxypropyl, Chlormethyl, Dichlormethyl, Trichlormethyl, Trifluormethyl, 2-Chlorethyl, 2,2,2-Trichlorethyl, 2-Bromethyl, 2,2,2-Tribromethyl, 3-Chlorpropyl, Cyclopropyl, Cyclopentyl, Cyclohexyl, Cycloheptyl oder für gegebenenfalls ein- bis dreifach, gleich oder verschieden durch Fluor, Chlor, Brom, Methyl, Ethyl, Methoxy, Ethoxy, Cyano oder Trifluormethyl substituiertes Benzyl oder Phenylethyl steht, oder für ein Äquivalent eines Natrium-, Kalium-, Calcium-, Magnesium-, Barium- oder Ammoniumions der Formel

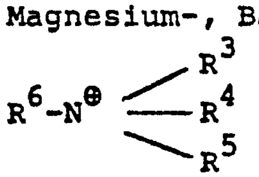

steht, wobei $R^3$, $R^4$, $R^5$ und $R^6$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, Cyclopentyl, Cyclohexyl oder Benzyl stehen und

$R^1$ und $R^2$ gemeinsam mit dem Stickstoffatom, an welches sie gebunden sind, für folgende gegebenenfalls durch Methyl, Methoxy und/oder Chlor substituierte heterocyclischen Reste stehen: 1,2,4-Triazol-1-yl, Imidazol-1-yl und Pyrazol-1-yl.

Im einzelnen seien außer den bei den Herstellungsbeispielen genannten Verbindungen, die folgenden N-Methyl-4-pyridone der allgemeinen Formel (I) genannt:

Tabelle 1:

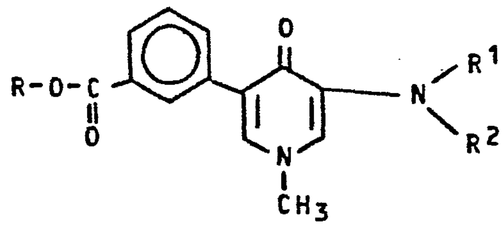

(I)

| R | 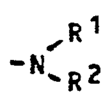 | R | 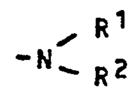 |
|---|---|---|---|
| H | 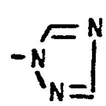 | $CH_3(CH_2)_3-CH(CH_3)-CH_2-$ | 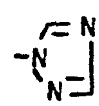 |
| $CH_3$ | 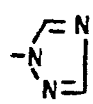 | $(CH_3)_2CH-CH_2-CH(CH_3)-$ | 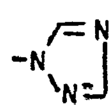 |
| $C_2H_5$ | 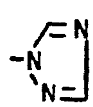 | $(CH_3)_3C-$ | 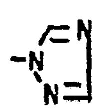 |

Tabelle 1 (Fortsetzung)

| R | 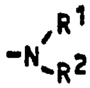 | R | 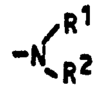 |
|---|---|---|---|
| n-$C_3H_7$ | 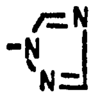 | Cl-$CH_2CH_2$- | 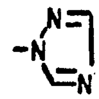 |
| i-$C_3H_7$ | 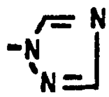 | | |
| t-$C_4H_9$ | 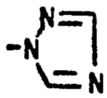 | 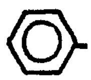 -$CH_2$- | 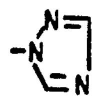 |
| $(CH_3)_2CH-CH_2CH_2$- | 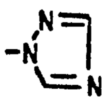 | 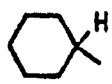 | 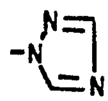 |
| $(C_2H_5)_2CH-CH_2$- | 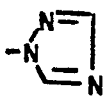 | $Na^{\oplus}$ | 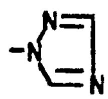 |
| $CH_3-(CH_2)_5$- | 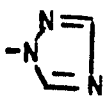 | $K^{\oplus}$ | 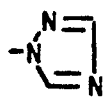 |
| $CH_3(CH_2)_3CH(C_2H_5)-CH_2$- | 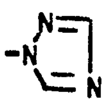 | $NH_4^{\oplus}$ | 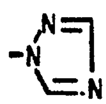 |
| $H_2N^{\oplus}(i-C_3H_7)_2$ | 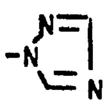 | n-$C_4H_9$ | 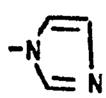 |
| 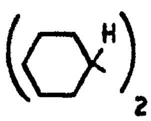 2 $NH_4^{\oplus}$ | 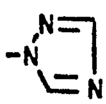 | i-$C_4H_9$ | 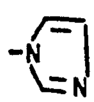 |
| 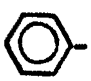 -$CH_2-N^{\oplus}(CH_3)_3$ | 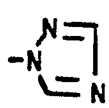 | s-$C_4H_9$- | 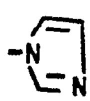 |
| $(C_2H_5)_3NH^{\oplus}$ | 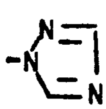 | t-$C_4H_9$ | 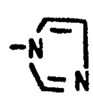 |

Tabelle 1 (Fortsetzung)

| R | $-N$ $R^1$ $R^2$ | R | $-N$ $R^1$ $R^2$ |
|---|---|---|---|
| $n-C_4H_9$ | 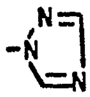 | $CH_3OCH_2CH_2-$ | 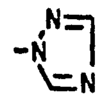 |
| $i-C_4H_9$ | 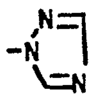 | $CH_2=CH-CH_2-$ | 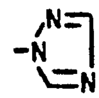 |
| $s-C_4H_9$ | 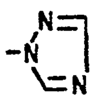 | $HC\equiv C-CH_2-$ | 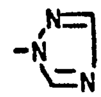 |
| $n-C_3H_7-NH_3^{\oplus}$ | 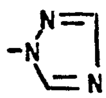 | $n-C_6H_{13}$ | 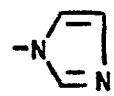 |
| $H_2N^{\oplus}(i-C_4H_9)_2$ | 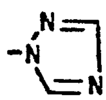 | $Cl-CH_2CH_2-$ | 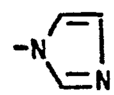 |
| $(n-C_3H_7)_2NH_2^{\oplus}$ | 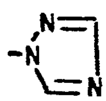 | $CH_3O-CH_2CH_2-$ | 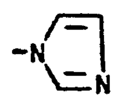 |
| $(CH_3)_2NH_2^{\oplus}$ | 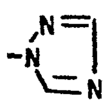 | $CH_2=CH-CH_2-$ | 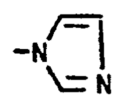 |
| $(C_2H_5)_2NH_2^{\oplus}$ | 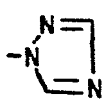 | $HC\equiv C-CH_2-$ | 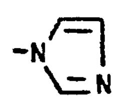 |
| $(n-C_4H_9)_2NH_2^{\oplus}$ | 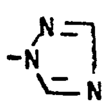 | 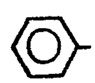 $-CH_2-$ | 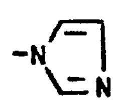 |
| $[CH_3(CH_2)_3-CH-CH_2-]_2NH_2^{\oplus}$<br>$C_2H_5$ | 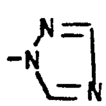 | 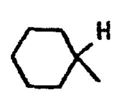 | 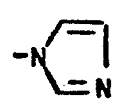 |

Tabelle 1 (Fortsetzung)

| R | 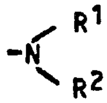 | R | 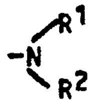 |
|---|---|---|---|
| H | 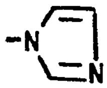 | $Na^{\oplus}$ | 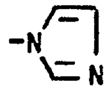 |
| $CH_3$ | 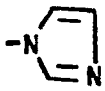 | $K^{\oplus}$ | 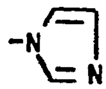 |
| $C_2H_5$ | 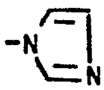 | $NH_4^{\oplus}$ | 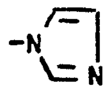 |
| $n-C_3H_7$ | 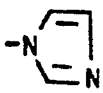 | $(C_2H_5)_3NH^{\oplus}$ | 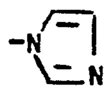 |
| $i-C_3H_7$ | 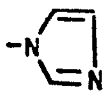 | $(i-C_3H_7)_2NH_2^{\oplus}$ | 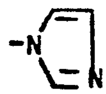 |
| $(CH_3)_2NH_2^{\oplus}$ | 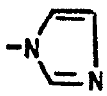 | $s-C_4H_9$ | 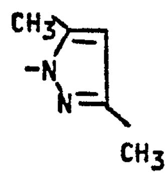 |
| H | 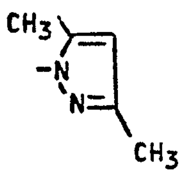 | $t-C_4H_9$ | 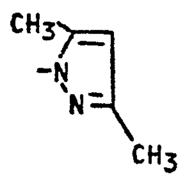 |
| $CH_3$ | 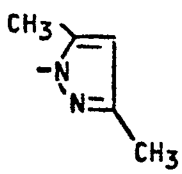 | $(CH_3)_2CHCH_2CH_2-$ | 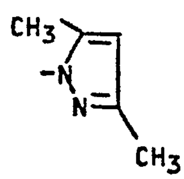 |

Tabelle 1 (Fortsetzung)

| R | 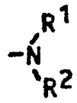 | R | 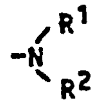 |
|---|---|---|---|
| $C_2H_5$ | 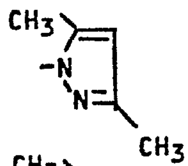 | $(C_2H_5)_2CHCH_2-$ | 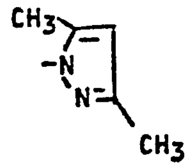 |
| $n-C_3H_7$ | 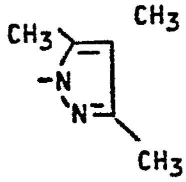 | $CH_3-(CH_2)_5-$ | 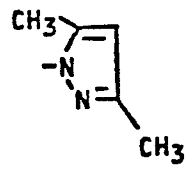 |
| $i-C_3H_7$ | 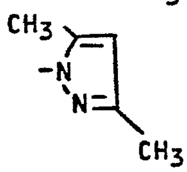 | $CH_3(CH_2)_3\underset{C_2H_5}{CH}-CH_2-$ | 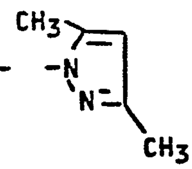 |
| $n-C_4H_9$ | 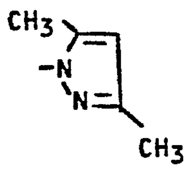 | $CH_3(CH_2)_3\overset{CH_3}{C}HCH_2-$ | 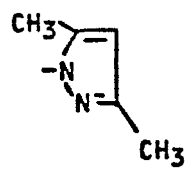 |
| $i-C_4H_9$ | 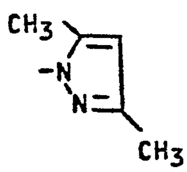 | $(CH_3)_2CH-CH_2\overset{CH_3}{C}H-$ | 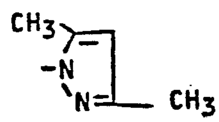 |
| $(CH_3)_3C-$ | 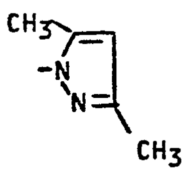 | $K^{\oplus}$ | 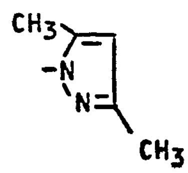 |
| $Cl-CH_2CH_2-$ | 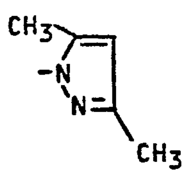 | $NH_4^{\oplus}$ | 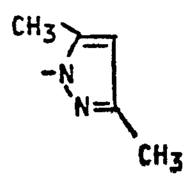 |

Tabelle 1 (Fortsetzung)

| R | $-N\begin{smallmatrix}R^1\\R^2\end{smallmatrix}$ | R | $-N\begin{smallmatrix}R^1\\R^2\end{smallmatrix}$ |
|---|---|---|---|
| $(CH_3)_2\overset{\oplus}{N}H_2$ | 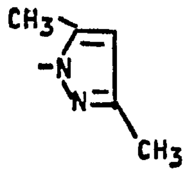 | $H_2\overset{\oplus}{N}(i\text{-}C_3H_7)_2$ | 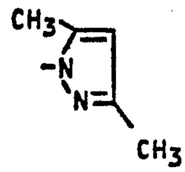 |
| $CH_3OCH_2CH_2-$ | 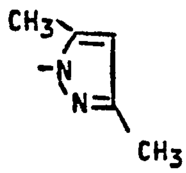 | 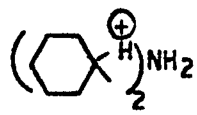 | 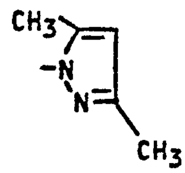 |
| $CH_2=CH-CH_2-$ | 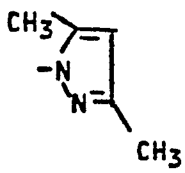 | 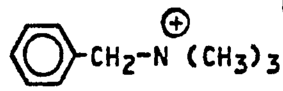 | 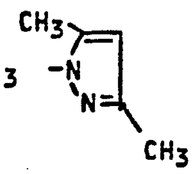 |
| $H-C\equiv C-CH_2-$ | 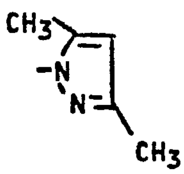 | $(C_2H_5)_3\overset{\oplus}{N}H$ | 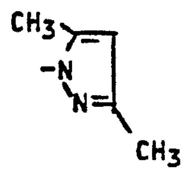 |
| 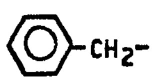 | 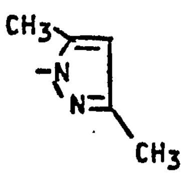 | $n\text{-}C_3H_7\text{-}\overset{\oplus}{N}H_3$ | 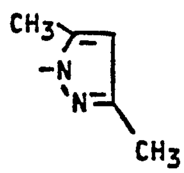 |
| 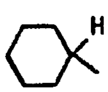 | 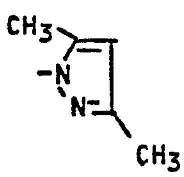 | $H_2\overset{\oplus}{N}(i\text{-}C_4H_9)_2$ | 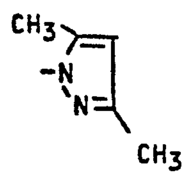 |
| $\overset{\oplus}{Na}$ | 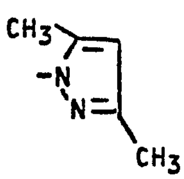 | $(n\text{-}C_3H_7)_2\overset{\oplus}{N}H_2$ | 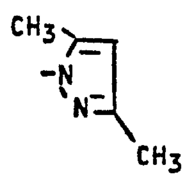 |

Tabelle 1 (Fortsetzung)

| R | 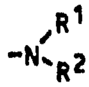 | R | 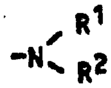 |
|---|---|---|---|
| $(C_2H_5)_2\overset{\oplus}{N}H_2$ | 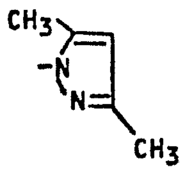 | s-$C_4H_9$ | 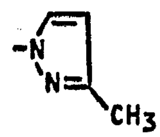 |
| $(n\text{-}C_4H_9)_2\overset{\oplus}{N}H_2$ | 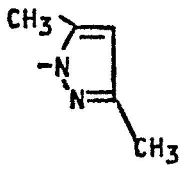 | t-$C_4H_9$ | 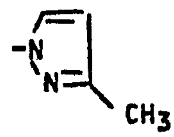 |
| $[CH_3(CH_2)_3\underset{C_2H_5}{\underset{\mid}{C}}HCH_2]_2\overset{\oplus}{N}H_2$ | 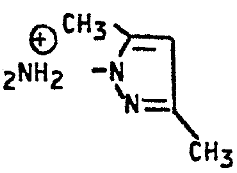 | n-$C_6H_{13}$ | 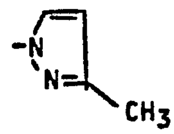 |
| H | 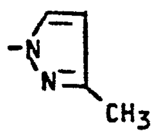 | $Cl-CH_2CH_2-$ | 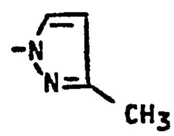 |
| $CH_3$ | 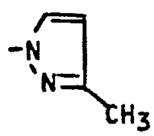 | $CH_3OCH_2CH_2-$ | 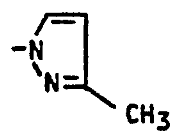 |
| $C_2H_5$ | 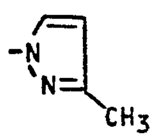 | $CH_2=CH-CH_2-$ | 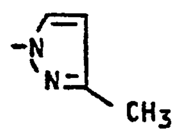 |
| n-$C_3H_7$ | 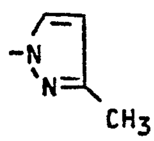 | $HC\equiv C-CH_2-$ | 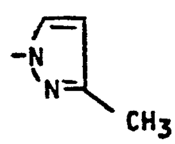 |
| | | 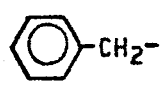 | 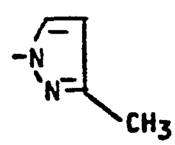 |

Tabelle 1 (Fortsetzung)

| R | 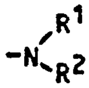 | R | 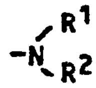 |
|---|---|---|---|
| $i-C_3H_7$ | 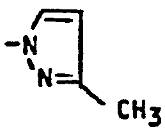 | 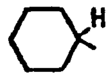 | 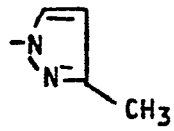 |
| $n-C_4H_9$ | 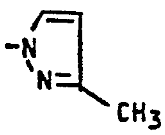 | $Na^{\oplus}$ | 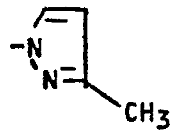 |
| $i-C_4H_9$ | 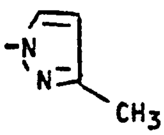 | $K^{\oplus}$ | 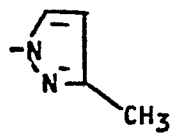 |
| $NH_4^{\oplus}$ | 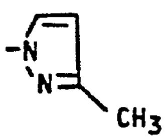 | $(C_2H_5)_3NH^{\oplus}$ | 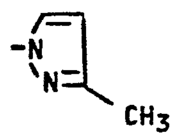 |
| $(i-C_3H_7)_2NH_2^{\oplus}$ | 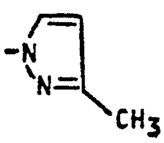 | $(CH_3)_2NH_2^{\oplus}$ | 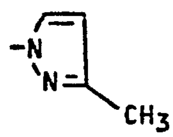 |

Verwendet man beispielsweise 1-Methyl-3-(pyrazol-1-yl)-5-(3-trifluormethylphenyl)-4-pyridon und konzentrierte Schwefelsäure als Ausgangsstoffe, so läßt sich der Reaktionsablauf des erfindugsgemäßen Verfahrens (a) durch das folgende Formelschema darstellen:

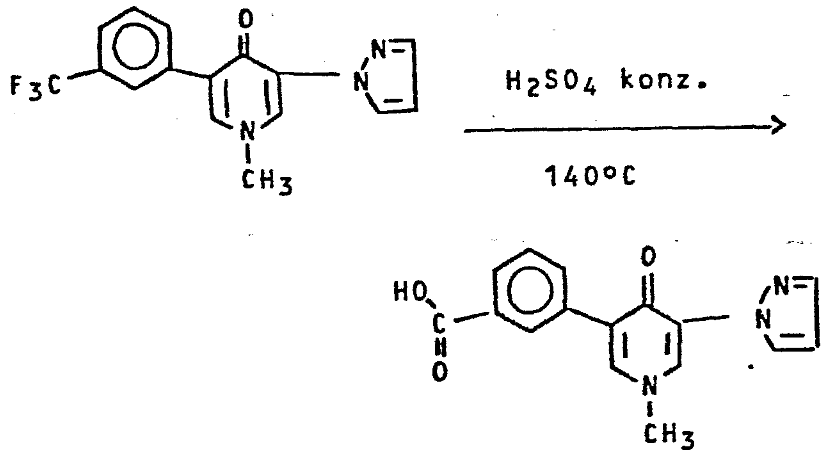


Verwendet man beispielsweise 5-(3-Carboxyphenyl)-1-methyl-3-(pyrazol-1-yl)-4-pyridon und Methanol als Ausgangsstoffe sowie Thionylchlorid als Katalysator, so läßt sich der Reaktionsablauf des erfindungsgemäßen Verfahrens (b) durch das folgende Formelschema darstellen:

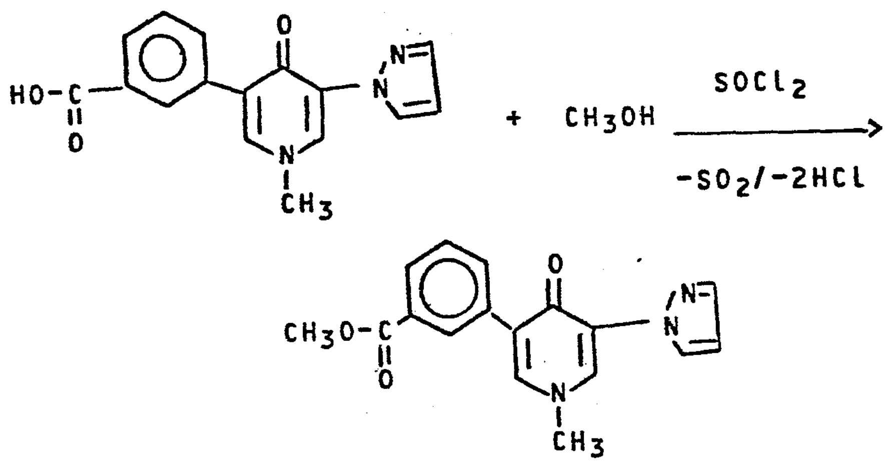


Verwendet man beispielsweise 5-(3-Methoxycarbonylphenyl)-1-methyl-3-(pyrazol-1-yl)-4-pyridon und n-Hexanol als

Ausgangsstoffe und Titantetraethylat als Katalysator, so läßt sich der Reaktionsablauf des erfindungsgemäßen Verfahrens (c) durch das folgende Formelschema darstellen:

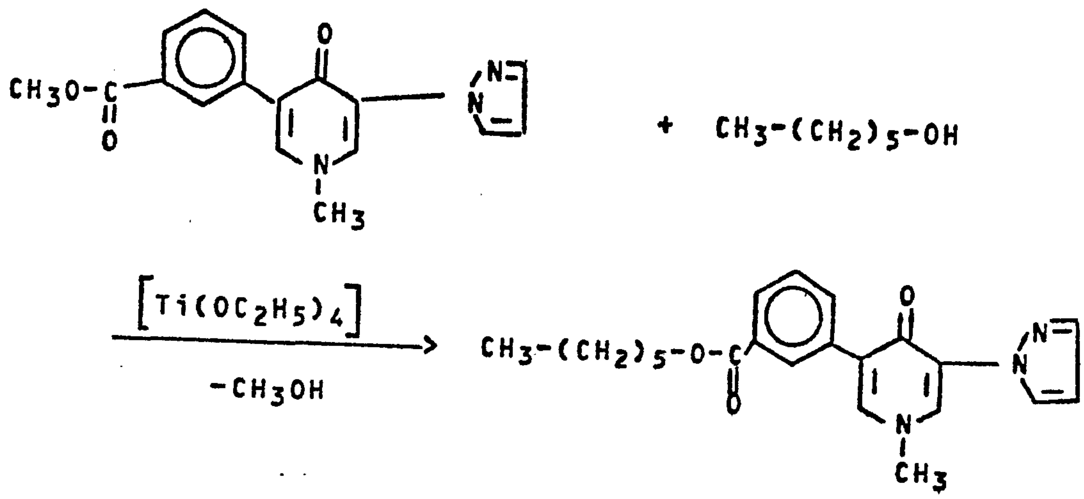


Die zur Durchführung des erfindungsgemäßen Verfahrens (a) als Ausgangsstoffe benötigten Trifluormethylphenyl-pyridone sind durch die Formel (II) allgemein definiert. In dieser Formel (II) stehen $R^1$ und $R^2$ vorzugsweise für diejenigen Substituenten, die bereits im Zusammenhang mit der Beschreibung der erfindungsgemäßen Stoffe der Formel (I) als bevorzugt für diese Substituenten genannt wurden.

Die Trifluormethylphenyl-pyridone der Formel (II) sind bekannt (vergl. z.B. EP-A2-0 073 999 bzw. US-PS 4 451 282).

Die zur Durchführung des erfindungsgemäßen Verfahrens (b) als Ausgangsstoffe benötigten Carbonsäuren sind durch die Formel (Ia) allgemein definiert. In dieser Formel

(Ia) stehen $R^1$ und $R^2$ ebenfalls vorzugsweise für diejenigen Reste, die bereits im Zusammenhang mit der Beschreibung der erfindungsgemäßen Stoffe der Formel (I) als bevorzugt für diese Substituenten genannt wurden.

Die Carbonsäuren der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Hydroxyverbindungen sind durch die Formel (III) allgemein definiert. In dieser Formel (III) steht R' vorzugsweise für jeweils geradkettiges oder verzweigtes Alkyl, Alkenyl oder Alkinyl mit jeweils bis zu 18 Kohlenstoffatomen, für geradkettiges oder verzweigtes Alkoxyalkyl mit jeweils bis zu 12 Kohlenstoffatomen in den einzelnen Alkylteilen, für geradkettiges oder verzweigtes Halogenalkyl mit bis zu 12 Kohlenstoffatomen und bis zu 18 gleichen oder verschiedenen Halogenatomen, für Cycloalkyl mit 3 bis 7 Kohlenstoffatomen oder für gegebenenfalls einfach oder mehrfach, gleich oder verschieden im Arylteil substituiertes, geradkettiges oder verzweigtes Aralkyl mit bis zu 4 Kohlenstoffatomen im Alkylteil und 6 bis 10 Kohlenstoffatomen im Arylteil steht, wobei als Substituenten in Frage kommen: Halogen, Cyano, Nitro, sowie jeweils geradkettiges oder verzweigtes Alkyl, Alkoxy oder Halogenalkyl mit jeweils bis zu 4 Kohlenstoffatomen und gegebenenfalls bis zu 9 gleichen oder verschiedenen Halogenatomen, für ein Äquivalent eines Alkali- oder Erdalkalimetallkations oder für ein

quartäres Ammoniumion der Formel 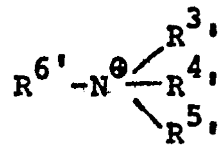 steht,

wobei $R^{3'}$, $R^{4'}$, $R^{5'}$ und $R^{6'}$ unabhängig voneinander jeweils für geradkettiges oder verzweigtes Alkyl mit bis zu 18 Kohlenstoffatomen, für Cycloalkyl mit 3 bis 7 Kohlenstoffatomen oder für geradkettiges oder verzweigtes Aralkyl mit bis zu 4 Kohlenstoffatomen im Alkylteil und 6-10 Kohlenstoffatomen im Arylteil stehen.

Die Hydroxyverbindungen der Formel (III) sind allgemein bekannte Verbindungen der organischen Chemie.

Die zur Durchführung des erfindungsgemäßen Verfahrens (b) alternativ als Ausgangstoffe benötigten Amine sind durch die Formel (IV) allgemein definiert. In dieser Formel (IV) stehen $R^3$, $R^4$ und $R^5$ vorzugsweise unabhängig voneinander jeweils für Wasserstoff, für geradkettiges oder verzweigtes Alkyl mit bis zu 18 Kohlenstoffatomen, für Cycloalkyl mit 3 bis 7 Kohlenstoffatomen oder für geradkettiges oder verzweigtes Aralkyl mit bis zu 4 Kohlenstoffatomen im Alkylteil und 6 bis 10 Kohlenstoffatomen im Arylteil.

Die Amine der Formel (IV) sind ebenfalls allgemein bekannte Verbindungen der organischen Chemie.

Die zur Durchführung des erfindungsgemäßen Verfahrens (c) als Ausgangsstoffe benötigten Ester sind durch die Formel (Ib) allgemein definiert. In dieser Formel (Ib) stehen $R^1$ und $R^2$ ebenfalls vorzugsweise für diejenigen Reste, die bereits bei der Beschreibung der erfindungsgemäßen Stoffe der Formel (I) als bevorzugt für diese Substituenten genannt wurden. R'' steht vorzugsweise für Methyl, Ethyl, n- oder i- Propyl.

Die Ester der Formel (Ib) sind erfindungsgemäße Stoffe und erhältlich mit Hilfe des erfindungsgemäßen Verfahrens (b).

Die zur Durchführung des erfindungsgemäßen Verfahrens (c) weiterhin als Ausgangsstoffe benötigten Alkohole sind durch die Formel (IIIa) allgemein definiert. In dieser Formel (IIIa) steht R''' vorzugsweise für jeweils geradkettiges oder verzweigtes Alkyl, Alkenyl oder Alkinyl mit jeweils 4 bis 18 Kohlenstoffatomen, für geradkettiges oder verzweigtes Alkoxyalkyl mit jeweils bis zu 12 Kohlenstoffatomen in den einzelnen Alkylteilen, für geradkettiges oder verzweigtes Halogenalkyl mit bis zu 12 Kohlenstoffatomen und bis zu 18 gleichen oder verschiedenen Halogenatomen, für Cycloalkyl mit 3 bis 7 Kohlenstoffatomen oder für gegebenenfalls einfach oder mehrfach, gleich oder verschieden im Arylteil substituiertes, geradkettiges oder verzweigtes Aralkyl mit bis zu 4 Kohlenstoffatomen im Alkylteil und 6 bis 10 Kohlenstoffatomen im Arylteil, wobei als Substituenten in Frage kommen: Halogen, Cyano, Nitro, sowie jeweils geradkettiges oder verzweigtes Alkyl, Alkoxy oder Halogenalkyl mit jeweils bis zu 4 Kohlenstoffatomen und gegebenenfalls bis zu 9 gleichen oder verschiedenen Halogenatomen.

Die Alkohole der Formel (IIIa) sind ebenfalls allgemein bekannte Verbindungen der organischen Chemie.

Das erfindungsgemäße Verfahren (a) wird vorzugsweise ohne Verdünnungsmittel in Substanz durchgeführt.

Die Reaktionstemperaturen können bei der Durchführung des erfindungsgemäßen Verfahrens (a) in einem größeren Bereich variiert werden. Im allgemeinen arbeitet man bei Temperaturen zwischen 20°C und 200°C, vorzugsweise bei Temperaturen zwischen 50°C und 150°C.

Zur Durchführung des erfindungsgemäßen Verfahrens (a) setzt man pro Mol Trifluormethylphenyl-pyridon der Formel (II) im allgemeinen 1,0 bis 15,0 Mol, vorzugsweise 1,0 bis 10,0 Mol an konzentrierter Schwefelsäure ein.

Die Reaktionsmischung wird bei der erforderlichen Temperatur bis zum Ende der Gasentwicklung gerührt, mit Wasser verdünnt und anschließend für einige Stunden unter Rückfluß erhitzt.

Der aus der erkalteten Reaktionsmischung durch Filtration gewonnene Niederschlag wird zur Entfernung anhaftender Schwefelsäure in wäßrig-alkoholischer Lösung mit Bariumhydroxid neutralisiert und nach Abfiltrieren des ausgefallenen Bariumsulfats mit Kohlensäure wiederausgefällt, abgesaugt und getrocknet.

Als Verdünnungsmittel zur Durchführung des erfindungsgemäßen Verfahrens (b) kommen organische Lösungsmittel in Frage.

Vorzugsweise verwendet man Alkohole, wie Methanol, Ethanol, n- oder Isopropanol.

Das erfindungsgemäße Verfahren (b) wird gegebenenfalls (im Fall der Veresterungsreaktion) in Gegenwart eines Katalysators durchgeführt. Als solche kommen alle üblicherweise für Veresterungsreaktionen verwendeten Katalysatoren infrage. Hierzu gehören z. B. Protonensäuren, wie beispielsweise Schwefelsäure oder p-Toluolsulfonsäure, Lewis-Säuren, wie beispielsweise Bortrifluorid oder Säurehalogenidbildner, wie Phosphorpentachlorid, Phosphortribromid oder Thionylchlorid.

Die Reaktionstemperaturen können bei der Durchführung des erfindungsgemäßen Verfahrens (b) ebenfalls in einem größeren Bereich variiert werden. Im allgemeinen arbeitet man bei Temperaturen zwischen 0°C und +150°C, vorzugsweise bei Temperaturen zwischen +20°C und +100°C.

Zur Durchführung des erfindungsgemäßen Verfahrens (b) setzt man pro Mol an Carbonsäure der Formel (Ia) im allgemeinen 1,0 bis 30,0 Mol, vorzugsweise 1,0 bis 20,0 Mol an Hydroxyverbindung der Formel (III) und gegebenenfalls 0,01 bis 2,0 Mol, vorzugsweise 0,1 bis 1,0 Mol an Katalysator ein. Die Reaktionsdurchführung, Aufarbeitung und Isolierung der N-Methyl-4-pyridone der Formel (I) erfolgt nach bekannten und üblichen Verfahren.

Als Verdünnungsmittel zur Durchführung des erfindungsgemäßen Verfahrens (c) kommen inerte organische Lösungsmittel in Frage. Vorzugsweise verwendet man Ether, wie

beispielsweise Tetrahydrofuran oder Dioxan. Es ist jedoch auch möglich, den als Reaktionspartner eingesetzten Alkohol der Formel (IIIa) in entsprechendem Überschuß als Verdünnungsmittel zu verwenden.

Als Katalysatoren für das erfindungsgemäße Verfahren (c) kommen insbesondere saure oder basische Katalysatoren in Frage. Besonders vorteilhaft verwendet man Lewis-Säuren, wie beispielsweise Bortrifluorid oder Titantetraethylat oder Alkoholate mit Natrium oder Kalium als Gegenion des entsprechenden zur Umesterung eingesetzten Alkohols der Formel (IIIa). Es ist auch möglich, anorganische Zeolithe zu verwenden, die den freiwerdenden niedermolekularen Alkohol binden und somit aus dem Reaktionsgleichgewicht entfernen.

Die Reaktionstemperaturen können bei der Durchführung des erfindungsgemäßen Verfahrens (c) ebenfalls in einem größeren Bereich variiert werden. Im allgemeinen arbeitet man bei Temperaturen zwischen 0°C und +120°C, vorzugsweise bei Temperaturen zwischen +30°C und +100°C.

Zur Durchführung des erfindungsgemäßen Verfahrens (c) setzt man pro Mol an Ester der Formel (Ib) im allgemeinen 1,0 bis 30 Mol, vorzugsweise 1,0 bis 20,0 Mol an Alkohol der Formel (IIIa) und 0,01 bis 2,0 Mol, vorzugsweise 0,1 bis 1,0 Mol an Katalysator ein. Die Reaktionsdurchführung, Aufarbeitung und Isolierung der Reaktionsprodukte erfolgt nach allgemein üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe lassen sich mit besonders gutem Erfolg zur selektiven Bekämpfung grasartiger und breitblättriger Unkräuter in wichtigen Kulturpflanzen, wie beispielsweise Baumwolle, einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen

0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

In entsprechenden Aufwandmengen besitzen die erfindungsgemäßen Wirkstoffe darüberhinaus auch eine fungizide Wirksamkeit und lassen sich beispielsweise zur Bekämpfung von Reiskrankheiten, wie z. B. gegen den Erreger der Reisfleckenkrankheit (Pyricularia oryzae) einsetzen.

Die Herstellung und die Verwendung der erfindungsgemäßen Wirkstoffe geht aus den nachfolgenden Beispielen hervor.

## Herstellungsbeispiele:

### Beispiel 1:

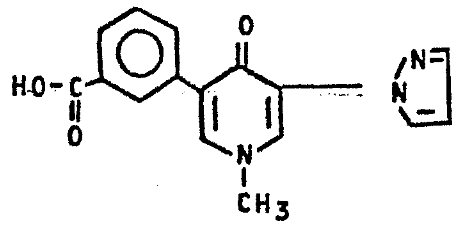

(Verfahren a)

150 g (0,47 Mol) 1-Methyl-3-(pyrazol-1-yl)-5-(3-trifluormethylphenyl)-4-pyridon werden tropfenweise unter Rühren mit 200 ml (3,74 Mol) konzentrierter Schwefelsäure versetzt, wobei die Temperatur auf 80°C bis 90°C ansteigt. Nach beendeter Zugabe erwärmt man unter Rühren bis zum Ende der Gasentwicklung (ca. 45 Minuten) auf 140°C, läßt dann auf ca. 40°C bis 50°C abkühlen, gibt in kleinen Portionen vorsichtig 200 ml Wasser zu und erhitzt nach beendeter Zugabe für ca. 60 Minuten auf Rückflußtemperatur. Der aus der erkalteten Reaktionsmischung ausgefallene Niederschlag wird abgesaugt, mit wenig Wasser gewaschen und bei 80°C getrocknet. Man erhält 148 g (ca. 80 % der Theorie) eines Feststoffes /5-(3-Carboxyphenyl)-1-methyl-3-(pyrazol-1-yl)-4-pyridon-Schwefelsäureaddukt (xn $H_2SO_4$)7, von dem 40 g (ca. 0,1 Mol) in einer Mischung aus 700 ml Wasser und 700 ml Ethanol bei 70°C gelöst werden. Zu dieser Lösung tropft man bis zum Erreichen von pH 7,5 eine wäßrige Bariumhydroxidlösung zu und saugt das ausgefallene Bariumsulfat über Kieselgur ab. Das Filtrat wird bei Raumtemperatur mit festem Kohlendioxid versetzt, bis bei einem pH-Wert von 5,5 die freie

Säure ausfällt, welche abgesaugt, mit wenig kaltem Wasser gewaschen und getrocknet wird. Man erhält 11,5 g (37 % der Theorie) an 5-(3-Carboxyphenyl)-1-methyl-3-(pyrazol-1-yl)-4-pyridon vom Schmelzpunkt 280°C.

Beispiel 2:

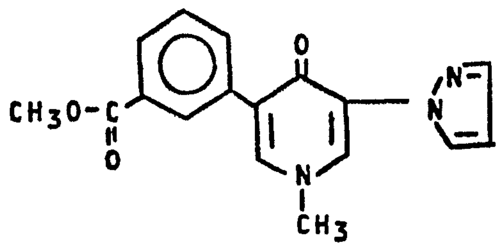

(Verfahren b)

30 g (0,09 Mol) 5-(3-Carboxyphenyl)-1-methyl-3-(pyrazol-1-yl)-4-pyridon-Schwefelsäureaddukt in 100 ml Methanol werden bei 0°C bis 10°C tropfenweise mit 24 ml (0,33 Mol) Thionylchlorid versetzt; nach beendeter Zugabe wird 12 Stunden bei Raumtemperatur gerührt, im Vakuum eingeengt, das verbleibende Öl in 100 ml Wasser aufgenommen, mit wäßriger Natriumcarbonatlösung schwach alkalisch eingestellt und dreimal mit jeweils 100 ml Dichlormethan extrahiert. Die vereinigten organischen Phasen werden mit Wasser gewaschen, über Natriumsulfat getrocknet und im Vakuum eingeengt. Die zurückbleibenden Kristalle werden mit Ether digeriert, abgesaugt und getrocknet. Man erhält 20 g (91 % der Theorie) an 5-(3-Methoxycarbonylphenyl)-1-methyl-3-(pyrazol-1-yl)-4-pyridon vom Schmelzpunkt 144°C.

Beispiel 3:

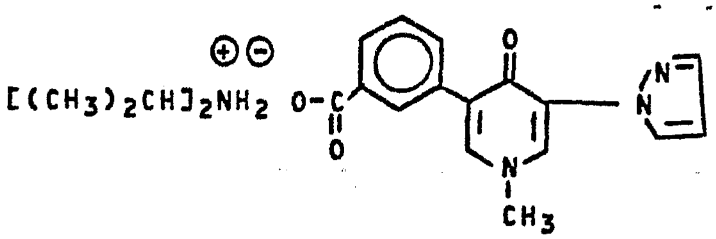

(Verfahren b)

3 g (0,01 Mol) 5-(3-Carboxyphenyl)-1-methyl-3-(pyrazol-1-yl)-4-pyridon und 1,425 ml (0,01 Mol) Diisopropylamin in 50 ml Ethanol werden eine Stunde bei Raumtemperatur gerührt und anschließend zur Trockne eingeengt. Man erhält 2 g (50 % der Theorie) an 3-/1-Methyl-5-(pyrazol-1-yl)-4-pyridon-3-yl7-benzoesäure-Diisopropylammonium-Salz vom Schmelzpunkt 174-175°C.

Beispiel 4:

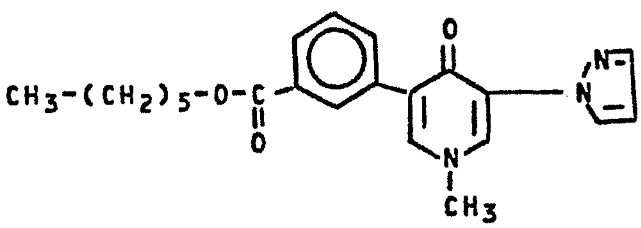

(Verfahren c)

5 g (0,016 Mol) 5-(3-Methoxycarbonylphenyl)-1-methyl-3-(pyrazol-1-yl)-4-pyridon und 16,32 g (0,16 Mol) n-Hexanol werden zusammen mit 3,64 g (0,016 Mol) Titantetraethylat in 50 ml absolutem Tetrahydrofuran 12 Stunden über einen mit 4Å-Zeolith gefüllten Soxhlet-Extraktor unter Rückfluß extrahiert. Die Reaktionsmischung wird zur Trockne eingeengt, in Wasser aufgenommen und dreimal mit Dichlormethan extrahiert; die vereinigten organischen Phasen

werden über Natriumsulfat getrocknet und im Vakuum eingeengt. Das verbleibende Öl wird chromatographisch über eine Kieselgelsäule (Laufmittel: Cyclohexan/Essigester 1:1) gereinigt und aus Ether kristallisiert. Man erhält 1,3 g (21,4 % der Theorie) an 5-(3-n-Hexyloxycarbonylphenyl)-1-methyl-3-(pyrazol-1-yl)-4-pyridon vom Schmelzpunkt 96-97°C.

Beispiel 5:

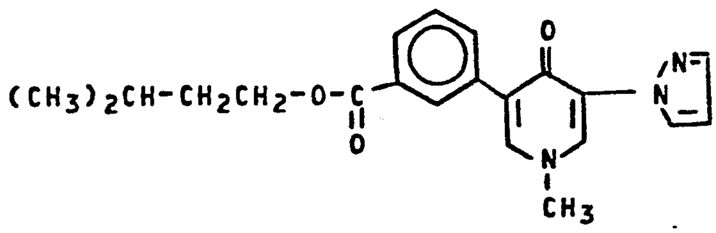

(Verfahren c)

Zu einer Lösung von 0,4 g (0,016 Mol) Natrium in 100 ml 3-Methyl-1-butanol gibt man 5 g (0,016 Mol) 5-(3-Methoxycarbonylphenyl)-1-methyl-3-(pyrazol-1-yl)-4-pyridon, erhitzt 12 Stunden unter Rückfluß, engt anschließend zur Trockne ein, nimmt den Rückstand in Wasser auf und extrahiert mehrfach mit Dichlormethan. Die vereinigten organischen Phasen werden über Natriumsulfat getrocknet und im Vakuum vom Lösungsmittel befreit. Das zurückbleibende Öl wird über eine Kieselgelsäule (Laufmittel: Cyclohexan/Essigester 1:1) chromatographiert und anschließend aus Ether kristallisiert.

Man erhält 1,2 g (22 % der Theorie) an 5-[3-(3-Methylbutoxycarbonyl)-phenyl]-1-methyl-3-(pyrazol-1-yl)-4-pyridon vom Schmelzpunkt 115-118°C.

In entsprechender Weise und gemäß den allgemeinen Herstellungsangaben erhält man die folgenden N-Methyl-4-pyridone der allgemeinen Formel (I):

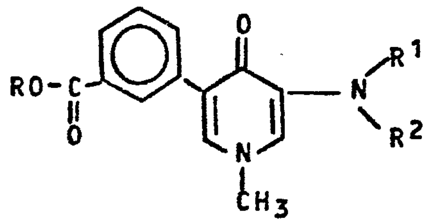

(I)

Tabelle 2:

| Bsp.Nr. | R | 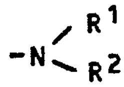 | Schmelzpunkt (°C) |
|---|---|---|---|
| 6 | $C_2H_5$ | 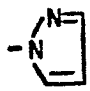 | 144 |
| 7 | $(CH_3)_2CH-$ | 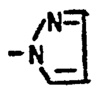 | 122 |
| 8 | $CH_3-(CH_2)_3-$ | 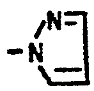 | 96-98 |
| 9 | $(CH_3)_3C-$ | 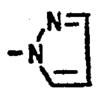 | 75 |
| 10 | $(CH_3)_2CH-CH_2-$ | 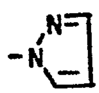 | 118-120 |
| 11 | $(C_2H_5)_2CH-CH_2-$ | 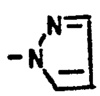 | 95-98 |
| 12 | $ClCH_2CH_2-$ | 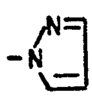 | 134-135 |
| 13 | $CH_3$<br>$CH_3(CH_2)_2CH-CH_2-$ | 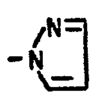 | 63-65 |
| 14 | 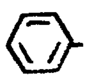 $-CH_2-$ | 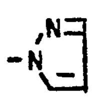 | 102-105 |

Tabelle 2 (Fortsetzung):

| Bsp.Nr. | R | $-N{<}^{R^1}_{R^2}$ | Schmelz-punkt (°C) |
|---|---|---|---|
| 15 | 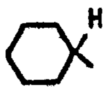 | 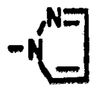 | 118-120 |
| 16 | $HC\equiv C-CH_2-$ | 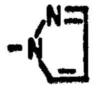 | 110-112 |
| 17 | $(CH_3)_3C-CH_2-$ | 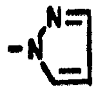 | 122-123 |
| 18 | $(CH_3)_2CH-CH_2\underset{CH_3}{\underset{\mid}{C}}H-$ | 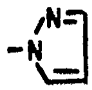 | $n_D^{35}$=1,5929 |
| 19 | $CH_2=CH-CH_2-$ | 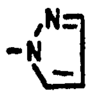 | 75 |
| 20 | $CH_3O-CH_2CH_2-$ | 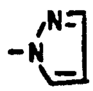 | 96 |
| 21 | $CH_3-(CH_2)_2\overset{\oplus}{N}H_3$ | 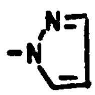 | 105-108 |
| 22 | $[(CH_3)_2CH-CH_2]_2\overset{\oplus}{N}H_2$ | 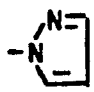 | 80-81 |
| 23 | $[CH_3(CH_2)_2]_2\overset{\oplus}{N}H_2$ | 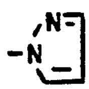 | 120-122 |
| 24 | $(C_2H_5)_3\overset{\oplus}{N}H$ | 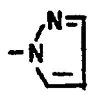 | Kristallbrei |
| 25 | $(CH_3)_2\overset{\oplus}{N}H_2$ | 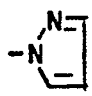 | 118-120 |
| 26 | $[CH_3(CH_2)_3\underset{C_2H_5}{\underset{\mid}{C}}H-CH_2-]_2\overset{\oplus}{N}H_2$ | 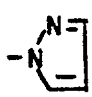 | Kristallbrei |

Tabelle 2 (Fortsetzung):

| Bsp.Nr. | R | 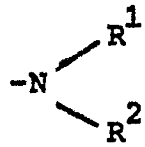 | Schmelz-punkt (°C) |
|---|---|---|---|
| 27 | $(C_2H_5)_2NH_2^{\oplus}$ | 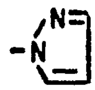 | 93 |
| 28 | $[CH_3(CH_2)_3-]_2NH_2^{\oplus}$ | 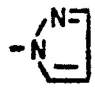 | 150-153 |
| 29 | $CH_3(CH_2)_3CHCH_2-$<br>$C_2H_5$ | 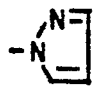 | 90-91 |
| 30 | 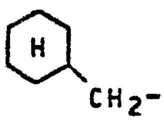 | 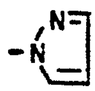 | 158-160°C |
| 31 | 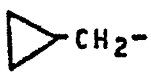 | 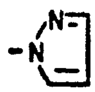 | 126 |
| 32 | 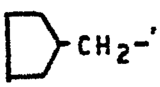 | 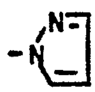 | |
| 33 | $Na^{\oplus}$ | 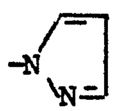 | 155 |
| 34 | 1/2 $Ba^{2\oplus}$ | 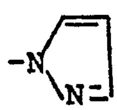 | >330 |
| 35 | $CH_3$ | 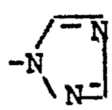 | 158-160 |

Tabelle 2 (Fortsetzung)

| Bsp.Nr. | R | 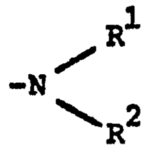 | Schmelzpunkt (°C) |
|---|---|---|---|
| 36 | $CH_3$ | 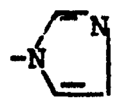 | Öl |
| 37 | $(CH_3)_2CH-CH_2-CH_2-$ | 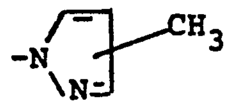 | Öl |
| 38 | $(CH_3)_2CH-CH_2-CH_2-$ | 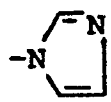 | Öl |
| 39 | $(C_2H_5)_2CH-CH_2-$ | 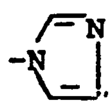 | Öl |
| 40 | $(CH_3)_2CH-CH_2-CH_2-$ | 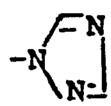 | 160-161 |
| 41 | $(C_2H_5)_2CH-CH_2-$ | 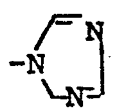 | 105 |

Tabelle 2 (Fortsetzung)

| Bsp.Nr. | R | 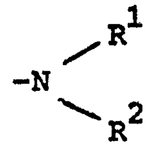 | Schmelzpunkt (°C) |
|---|---|---|---|
| 42 | $CH_3-(CH_2)_3-$ | 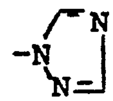 | 166 |
| 43 | $(CH_3)_2CH-CH_2-CH_2-$ | 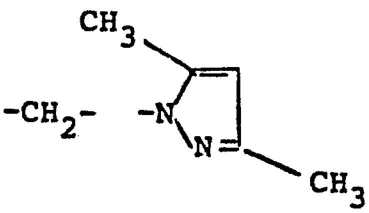 | Öl |
| 44 | $CH_3-(CH_2)_3-$ | 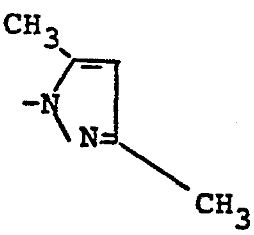 | Öl |
| 45 | $(C_2H_5)_2CH-CH_2-$ | 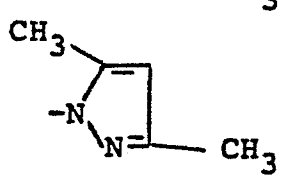 | Öl |

Anwendungsbeispiele:

In dem folgenden Anwendungsbeispiel wurde die nachstehend aufgeführte Verbindung als Vergleichssubstanz eingesetzt:

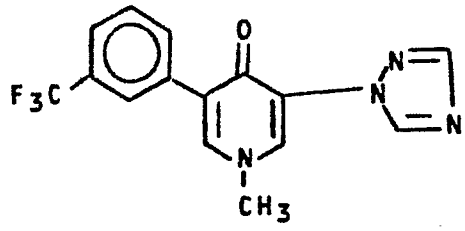

(A)

1-Methyl-3-(1,2,4-triazol-1-yl)-5-(3-trifluormethylphenyl)-4-pyridon (bekannt aus EP-A2-0 073 999 und US-PS 4 451 282).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigt in diesem Test z. B. die Verbindung gemäß dem Herstellungsbeispiel: 11.

Patentansprüche

1) N-Methyl-4-pyridone der allgemeinen Formel (I)

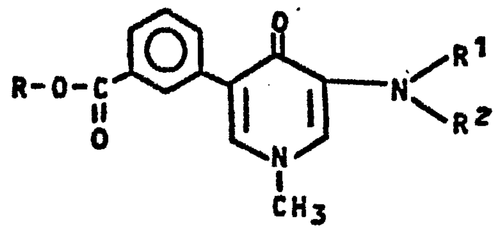

(I)

in welcher

R für Wasserstoff, Alkyl, Alkenyl, Alkinyl, Alkoxyalkyl, Halogenalkyl, Cycloalkyl, für gegebenenfalls substituiertes Aralkyl oder für ein Äquivalent eines Ammonium- oder Metallkations steht und

$R^1$ und $R^2$ gemeinsam mit dem Stickstoffatom, an welches sie gebunden sind, für einen gegebenenfalls substituierten Heterocyclus stehen.

2) N-Methyl-4-pyridone der allgemeinen Formel (I) gemäß Anspruch 1, dadurch gekennzeichnet, daß darin

R für Wasserstoff, für jeweils geradkettiges oder verzweigtes Alkyl, Alkenyl oder Alkinyl mit jeweils bis zu 18 Kohlenstoffatomen, für geradkettiges oder verzweigtes Alkoxyalkyl mit jeweils bis zu 12 Kohlenstoffatomen in den einzelnen Alkylteilen, für geradkettiges oder verzweigtes Halogenalkyl mit bis zu 12 Kohlenstoffatomen und bis zu 18 gleichen oder

verschiedenen Halogenatomen, für Cycloalkyl mit 3 bis 7 Kohlenstoffatomen oder für gegebenenfalls einfach oder mehrfach, gleich oder verschieden im Arylteil substituiertes, geradkettiges oder verzweigtes Aralkyl mit bis zu 4 Kohlenstoffatomen im Alkylteil und 6 bis 10 Kohlenstoffatomen im Arylteil steht, wobei als Substituenten infrage kommen: Halogen, Cyano, Nitro, sowie jeweils geradkettiges oder verzweigtes Alkyl, Alkoxy oder Halogenalkyl mit jeweils bis zu 4 Kohlenstoffatomen und gegebenenfalls bis zu 9 gleichen oder verschiedenen Halogenatomen, außerdem für ein Äquivalent eines Alkali- oder Erdalkalimetallkations oder für ein Ammoniumion der Formel

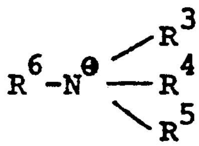

steht, wobei $R^3$, $R^4$, $R^5$ und $R^6$ unabhängig voneinander jeweils für Wasserstoff, für geradkettiges oder verzweigtes Alkyl mit bis zu 18 Kohlenstoffatomen, für Cycloalkyl mit 3 bis 7 Kohlenstoffatomen oder für geradkettiges oder verzweigtes Aralkyl mit bis zu 4 Kohlenstoffatomen im Alkylteil und 6 bis 10 Kohlenstoffatomen im Arylteil stehen,

und

$R^1$ und $R^2$ gemeinsam mit dem Stickstoffatom, an welches sie gebunden sind, für einen gegebenenfalls durch Alkyl oder Alkoxy mit jeweils bis zu 4 Kohlenstoffatomen oder Halogen substituierten 5-gliedrigen Heterocyclus mit 1 bis 3 Stickstoffatomen stehen.

3) N-Methyl-4-pyridone der allgemeinen Formel (I) gemäß Anspruch 1, dadurch gekennzeichnet, daß darin

R für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, n- oder i-Nonyl, n- oder i-Decyl, n- oder i-Dodecyl, Allyl, Butenyl, Pentenyl, Hexenyl, Propargyl, Butinyl, Pentinyl, Hexinyl, Methoxymethyl, Methoxyethyl, Ethoxyethyl, Isopropoxyethyl, Methoxypropyl, Chlormethyl, Dichlormethyl, Trichlormethyl, Trifluormethyl, 2-Chlorethyl, 2,2,2-Trichlorethyl, 2-Bromethyl, 2,2,2-Tribromethyl, 3-Chlorpropyl, Cyclopropyl, Cyclopentyl, Cyclohexyl, Cycloheptyl oder für gegebenenfalls ein- bis dreifach, gleich oder verschieden durch Fluor, Chlor, Brom, Methyl, Ethyl, Methoxy, Ethoxy, Cyano oder Trifluormethyl substituiertes Benzyl oder Phenylethyl steht, oder für ein Äquivalent eines Natrium-, Kalium-, Calcium-, Magnesium-, Barium- oder Ammoniumions der Formel

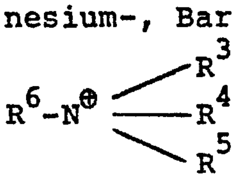

steht, wobei $R^3$, $R^4$, $R^5$ und $R^6$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, Cyclopentyl, Cyclohexyl oder Benzyl stehen und

$R^1$ und $R^2$ gemeinsam mit dem Stickstoffatom, an welches sie gebunden sind, für folgende gegebenenfalls durch Methyl, Methoxy und/oder Chlor substituierte heterocyclischen Reste stehen: 1,2,4-Triazol-1-yl, Imidazol-1-yl und Pyrazol-1-yl.

4) Verfahren zur Herstellung von N-Methyl-4-pyridonen der allgemeinen Formel (I)

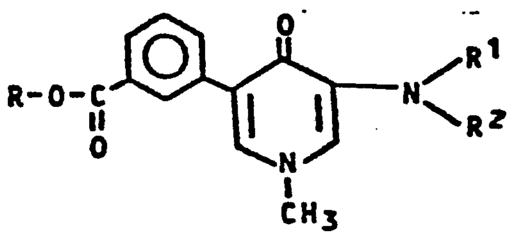

(I)

in welcher

R für Wasserstoff, Alkyl, Alkenyl, Alkinyl, Alkoxyalkyl, Halogenalkyl, Cycloalkyl, für gegebenenfalls substituiertes Aralkyl oder für ein Äquivalent eines Ammonium- oder Metallkations steht und

$R^1$ und $R^2$ gemeinsam mit dem Stickstoffatom, an welches sie gebunden sind, für einen gegebenenfalls substituierten Heterocyclus stehen,

dadurch gekennzeichnet, daß man

(a) Trifluormethylphenyl-pyridone der Formel (II),

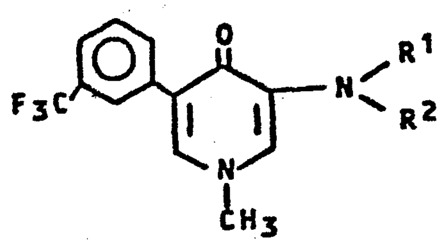

(II)

in welcher

$R^1$ und $R^2$ die oben angegebene Bedeutung haben,

mit konzentrierter Schwefelsäure verseift, oder daß man

(b) die nach Verfahren (a) erhältlichen Carbonsäuren der Formel (Ia),

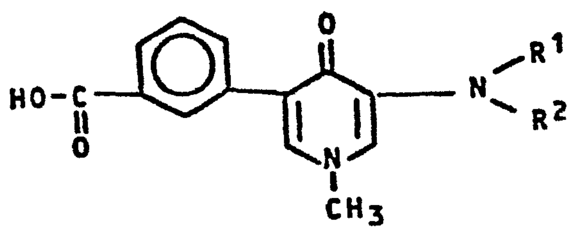

(Ia)

in welcher

$R^1$ und $R^2$ die oben angegebene Bedeutung haben,

mit Hydroxyverbindungen der Formel (III),

$$R' - OH \quad \text{(III)}$$

in welcher

R' für Alkyl, Alkenyl, Alkinyl, Alkoxyalkyl, Halogenalkyl, Cycloalkyl, für gegebenenfalls substituiertes Aralkyl oder für ein Äquivalent eines Metallkations oder quarternären Ammoniumions steht,

oder mit Aminen der Formel (IV),

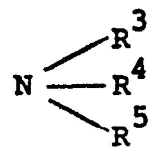

(IV)

in welcher

$R^3$, $R^4$ und $R^5$ unabhängig voneinander jeweils für Wasserstoff oder für Alkyl, Cycloalkyl oder Aralkyl stehen,

gegebenenfalls in Gegenwart eines Verdünnungsmittels und gegebenenfalls in Gegenwart eines Katalysators umsetzt, oder daß man

(c) die nach Verfahren (b) erhältlichen Ester der Formel (Ib)

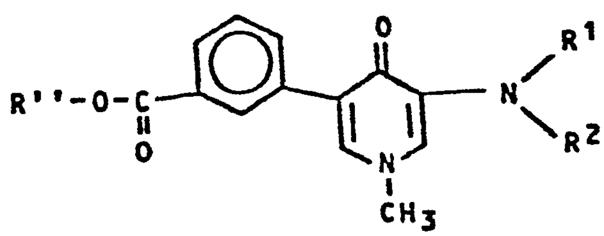

(Ib)

in welcher

R'' für niederes Alkyl steht

und

$R^1$ und $R^2$ die oben angegebene Bedeutung haben,

mit Alkoholen der Formel (IIIa)

$$R''' - OH \qquad (IIIa)$$

in welcher

R''' für Alkyl, Alkenyl, Alkinyl, Alkoxyalkyl, Halogenalkyl, Cycloalkyl oder für gegebenenfalls substituiertes Aralkyl steht,

gegebenenfalls in Gegenwart eines Verdünnungsmittels und gegebenenfalls in Gegenwart eines Katalysators umsetzt.

5) Herbizide Mittel, gekennzeichnet durch einen Gehalt an mindestens einem N-Methyl-4-pyridon der Formel (I) gemäß Anspruch 1 und 4.

6) Verfahren zur Bekämpfung von Unkraut, dadurch gekennzeichnet, daß man N-Methyl-4-pyridone der Formel (I) gemäß Anspruch 1 und 4 auf die Unkräuter oder ihren Lebensraum einwirken läßt.

7) Verwendung von N-Methyl-4-pyridonen der Formel (I) gemäß Anspruch 1 und 4 zur Bekämpfung von unerwünschtem Pflanzenwachstum.

8) Verfahren zur Herstellung von N-Methyl-4-pyridonen der Formel (I) gemäß Anspruch 1 und 4, dadurch gekennzeichnet, daß man N-Methyl-4-pyridone der Formel (I) gemäß Anspruch 1 und 4 mit Streckmitteln und/oder oberflächenaktiven Mitteln vermischt.

9) 5-/3-(2-Ethyl-butoxycarbonyl)-phenyl/-1-methyl-3-(pyrazol-1-yl)-4-pyridon der Formel

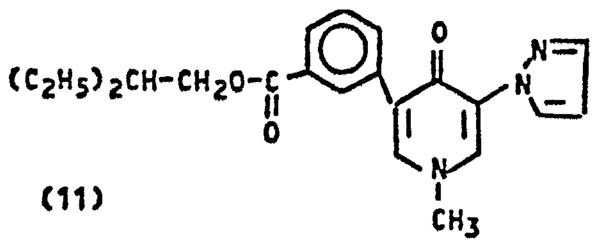

(11)

gemäß Anspruch 1.

Europäisches Patentamt

# EUROPÄISCHER RECHERCHENBERICHT

0171729
Nummer der Anmeldung

EP 85 10 9817

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X,Y D | EP-A-0 073 999 (BAYER)<br>* Patentansprüche 1-3,5-8; Seite 5, Zeilen 13-17 * | 1-9 | C 07 D 401/04<br>A 01 N 43/56<br>A 01 N 43/653<br>A 01 N 43/50 |
| Y | DE-A-2 537 753 (ELI LILLY)<br>* Seiten 1-12; Patentansprüche 1,3,8,10 * | 1-9 | |
| | | | RECHERCHIERTE SACHGEBIETE (Int Cl 4) |
| | | | C 07 D 401/00 |

Der vorliegende Recherchenbericht wurde für alle Patentansprüche erstellt

| Recherchenort | Abschlußdatum der Recherche | Prüfer |
|---|---|---|
| DEN HAAG | 24-10-1985 | THEUNS H.G. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82